# EUROPEAN PATENT SPECIFICATION

(11) **EP 2 522 271 B1**
(45) Date of publication and mention of the grant of the patent: **27.08.2014**
(21) Application number: 12167669.6
(22) Date of filing: 11.05.2012
(51) Int. Cl.: A61B 1/05

(54) **Twin camera endoscope**
Zwillingskamera-Endoskop
Endoscope de caméra jumelé

(30) Priority: 13.05.2011 US 201161485734 P; 09.04.2012 US 201213442009
(43) Date of publication of application: 14.11.2012
(73) Proprietor: Covidien LP, Mansfield, MA 02048 (US)
(72) Inventor: Viola, Frank, Sandy Hook, CT Connecticut 06482 (US)
(74) Representative: Soames, Candida Jane

(56) References cited:
- US-A- 5 474 519
- US-A1- 2008 027 279

## Description

### CROSS REFERENCE TO RELATED APPLICATION

The present application claims the benefit of and priority to U.S. Provisional Application Serial No.61/485,734, filed on May 13, 2011.

### BACKGROUND

### Technical Field

The present disclosure relates to surgical instruments, and more particularly, to a surgical camera assembly having a pair of cameras for three-dimensional viewing of an internal surgical site.

### Background of Related Art

Endoscopic surgical procedures are minimally invasive procedures in which operations are carried out within the body by using elongated instruments inserted through small entrance openings in the body. Minimally invasive procedures are desirable in that they allow for quicker recovery time and shorter hospital stays as compared to open surgical procedures. Minimally invasive procedures also leave minimal scarring (both internally and externally) and reduce patient discomfort during the recovery period. However, because the interior dimensions of the entrance openings into the body are necessarily small, only elongated, small diametered instrumentation may be used to access the internal surgical site.

During a typical minimally-invasive surgical procedure, a surgical camera, or endoscope, is inserted through an access opening in the body to permit the surgeon to view the internal site. Where three-dimensional viewing of the internal surgical site is desired, multiple cameras, or an endoscope including multiple cameras disposed thereon, are inserted into the surgical site through additional access openings formed within the body. Endoscopes including multiple cameras disposed thereon are advantageous in that only one access opening is required, however the access opening may need to be substantial in order to accommodate the endoscope therein, thus requiring that the access opening be sutured closed at the completion of the procedure. Providing multiple cameras, on the other hand, permits each camera to be inserted through a relatively smaller access opening, but adds the complication of having to maintain each of the cameras in fixed relation relative to one another.

US 5474519 discloses a surgical camera assembly as recited in the preamble of the independent claim.

### SUMMARY

In accordance with one embodiment of the present disclosure, a surgical camera assembly is provided as recited in claim 1. The surgical camera assembly includes a base having first and second spaced-apart legs extending distally therefrom. The legs are configured for positioning within an internal surgical site, are disposed in parallel orientation relative to one another, and are fixed in distance to one another and independently slidable in the distal direction relative to the base. Each leg defines a proximal end slidably engaged to the base and a free distal end. A first surgical camera is disposed within the first leg and is configured to produce a video image of the area extending distally and radially outwardly from the distal end of the first leg. A second surgical camera is disposed within the second leg and is configured to produce a video image of the area extending distally and radially outwardly from the distal end of the second leg. The video image produced by the first surgical camera and the video image produced by the second surgical camera are used in conjunction with one another to provide a three-dimensional video image of the internal surgical site.

In one embodiment, the first and second legs are spaced-apart by a distance of about 63,5 mm (2.5 inches), the average distance between an adult human's eyes. This embodiment allows, in a further embodiment, stereoscopic viewing.

In another embodiment, each of the legs defines a diameter of about 1mm to about 2mm to facilitate insertion of the legs through relatively small incisions in tissue.

In another embodiment, the surgical camera assembly further includes a pair of visualization goggles configured to be worn by a surgeon. The visualization goggles are coupled to the base, e.g., via a cable or wirelessly, and are configured to display the video images produced by each of the first and second cameras, thus providing the surgeon with a three-dimensional view of the internal surgical site.

In another embodiment, the visualization goggles are configured to display the video image produced by the first camera in front of a first eye of the surgeon and the video image produced by the second camera in front of a second eye of the surgeon.

In another embodiment, the surgical cameras may each include a lens, an image sensor, and control circuitry. The control circuitry of each of the surgical cameras may be disposed within the base of the surgical camera assembly.

A method of three-dimensionally visualizing an internal surgical site is described. The method includes providing a surgical camera assembly according to any of the embodiments above. The method further includes forming first and second spaced-apart incisions in tissue, inserting the first leg of the surgical camera assembly through the first incision and the second leg of the surgical camera assembly through the second incision such that the first and second legs are disposed within the internal surgical site. Thereafter, a first video image of the internal surgical site via the first surgical camera is produced and a second video image of the internal surgical site via the second surgical camera is produced. The method further includes viewing the first video image with one eye and viewing the second video image with a second eye to three-dimensionally visualize the internal surgical site.

### BRIEF DESCRIPTION OF THE DRAWINGS

Various embodiments of the subject instrument are described herein with reference to the drawings wherein:

Fig. 1 is front, perspective view of a surgical camera assembly provided in accordance with the present disclosure;

Fig. 2 is a longitudinal, cross-sectional view of a leg and a portion of a base of the surgical camera assembly of Fig. 1;

Fig. 3 is a perspective view of the surgical camera assembly of Fig. 1 shown coupled to a pair of visualization goggles; and

Fig. 4 is a cross-sectional view of the surgical camera assembly of Fig. 1 shown positioned within an internal surgical site.

### DETAILED DESCRIPTION

Embodiments of the present disclosure are described in detail with reference to the drawing figures wherein like reference numerals identify similar or identical elements. As used herein, the term "distal" refers to the portion that is being described which is further from a user, while the term "proximal" refers to the portion that is being described which is closer to a user.

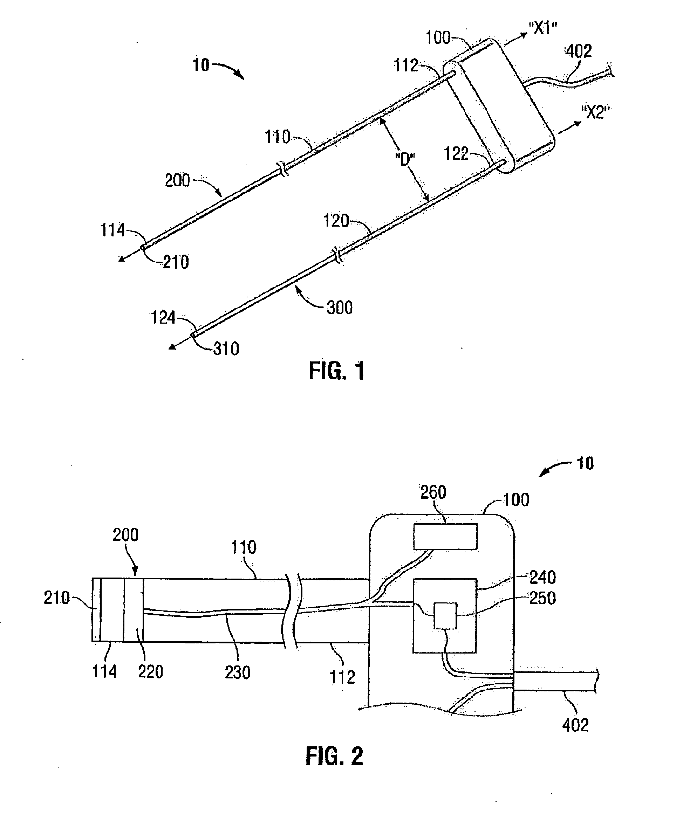

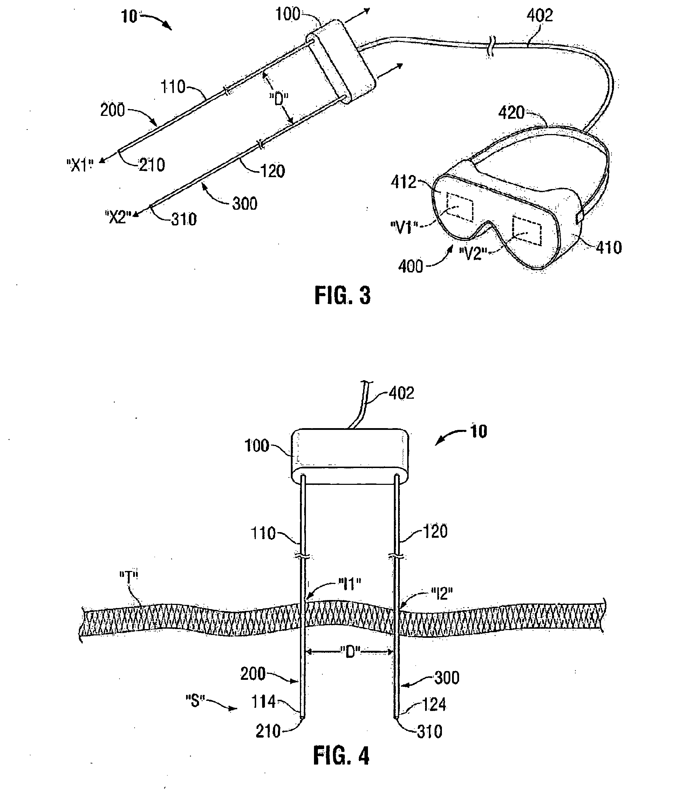

Referring now to Figs. 1-3, a surgical camera assembly provided in accordance with the present disclosure is shown generally identified by reference numeral 10. Surgical camera assembly 10 generally includes a base 100 having a pair of elongated legs 110, 120 extending distally therefrom. Legs 110, 120 are slidingly engaged to base 110 to allow movement in the proximal/distal axial direction, being a set distance apart at proximal ends 112, 122, respectively, thereof and extending therefrom to free distal ends 114, 124, respectively, thereof. More particularly, legs 110, 120 are slidingly disposed in parallel, spaced-apart relation relative to one another to define a fixed distance "D," e.g., about 63,5 mm (2.5 inches), therebetween along the full lengths thereof. As will be described in greater detail below, each leg 110, 120 is configured for insertion through an incision "I1," "I2," respectively, formed within tissue "T" (see Fig. 4) and includes a surgical camera 200, 300 disposed therein. Surgical cameras 200, 300 cooperate to provide three-dimensional, or stereoscopic imaging of an internal surgical site "S" (Fig. 4). Further, each leg 110, 120 may define a diameter of about 1mm to about 2mm, although other diameters are contemplated, to facilitate insertion of legs 110, 120 through relatively small incisions "I1," "I2," respectively, formed within tissue "T" (see Fig. 4).

With continued reference to Fig. 1, each leg 110, 120 defines a longitudinal axis "X1" and "X2," respectively, that, as a result of the parallel orientation of legs 110, 120, are parallel to one another. Each leg 110, 120 also includes a respective lens 210, 310 disposed at distal end 114, 124 thereof, that is configured to receive an image of the area extending distally from lenses 210, 310 along and radially outwardly from respective longitudinal axes "X1," "X2," of legs 110, 120, respectively. This configuration is employed such that the images received by lenses 210, 310 of surgical cameras 200, 300 of legs 110, 120, respectively, are of substantially the same area, but from different perspectives. In other words, the images receive by surgical cameras 200, 300 are offset relative to one another by a distance "D," the distance between lenses 116, 126 of surgical cameras 200, 300 of legs 110, 120, respectively. Legs 110, 120 may each further include an illumination source (not shown) configured to illuminate the internal surgical site "S" (Fig. 4) to facilitate visualization thereof.

As mentioned above, the distance "D" between legs 110, 120 and, thus, lenses 210, 310 of surgical cameras 200, 300, respectively, may be about 63,5 mm (2.5 inches), which corresponds to the approximate average distance between the eyes (measured from center of one pupil to the center of the other pupil) of an adult human. As will be described in greater detail below, configuring surgical camera assembly 10 such that legs 110, 120 are spaced-apart a distance "D" that is approximately equal to the distance between a human's eyes permits the surgeon to visualize the internal surgical site "S" (Fig. 4) in three-dimensions as if the surgeon were viewing the internal surgical site "S" (Fig. 4) directly with his/her own eyes, i.e., camera 200 of leg 110 produces an image similar to the image that would be seen by one eye of the surgeon, while the other camera 300 of leg 120 produces an image similar to the image that would be seen by the other eye of the surgeon. Cameras 200, 300 of legs 110, 120, respectively, are substantially similar to one another and, thus, only camera 200 will be described hereinbelow to avoid unnecessary repetition. Further, although one example of a camera 200 configured for use with surgical camera assembly 10 is described below, it is envisioned that any other suitable camera may be provided for use in conjunction with surgical camera assembly 10. As an alternative to cameras 200, 300, fiber optic bundles (not shown) may transmit the image.

Continuing with reference to Figs. 1-3, and to Fig. 2 in particular, surgical camera 200 includes an image sensor 220 disposed within leg 110 that receives the optical image projected thereon by lens 210. Image sensor 220 is configured to convert the optical image received from lens 210 into an electrical signal. Image sensor 220 may be a CCD image sensor, a CMOS image sensor, or any other suitable image sensor as is known in the art.

With continued reference to Fig. 2, image sensor 220 is electrically coupled to an insulated wire, or bundle of wires 230, that extends from image sensor 220 proximally though leg 110 and into base 100 of surgical camera assembly 10. The image sensor (not shown) of camera 300 (Fig. 1) disposed within leg 120 (Fig. 1) similarly includes wire(s) (not shown) that extend proximally through leg 120 (Fig. 1) and into base 100. Wire(s) 230 transmit the electrical signal produced by image sensor 220 through leg 110 to control circuitry 240 disposed within base 100. Wire(s) 230 may also be configured to transfer power to image sensor 230 from a battery 260 disposed within base 100 or from an external power source (not explicitly shown). The wire(s) (not shown) of camera 300 (Fig. 1) are similarly configured for transmitting the electrical signal and/or power between the image sensor (not shown) of camera 300 (Fig. 1) and control circuitry (not shown) associated therewith that is disposed within base 100. Control circuitry 240 (and the control circuitry (not shown) associated with camera 300) may include a processing component 250 that processes the electrical signal transmitted through wire(s) 230, e.g., converts the signal from analog to digital, and/or modulates the signal. Control circuitry 240 and the control circuitry (not shown) associated with camera 300 then each transmit their respective signals, e.g., the converted and/or modulated signals, to a display 400 via cable 402. Alternatively, control circuitry 240 (and the control circuitry (not shown) associated with camera 300) may be configured to wirelessly transmit, or broadcast the processed signal to a wireless receiver (not explicitly shown) associated with the display 400. Display 400, as will be described in greater detail below, may be a pair of visualization goggles 400. However, it is envisioned that any other suitable display, e.g., a standard video monitor (not shown) may also be provided. For use with a standard video monitor (not shown), the signals from control circuitry 240 and the control circuitry (not shown) of camera 300 (Fig. 1) are multiplexed or synthesized, either within base 100, or externally, to produce an electronically synthesized three-dimensional video image for display on the video monitor (not shown).

A can be appreciated, in embodiments where battery 260 is disposed within base 100 for powering cameras 200, 300, and where base 100 is configured to wirelessly transmit the signal(s) to display 400, surgical camera assembly 10 need not require any external cables or wires, i.e. surgical camera assembly 10 is fully wireless. Once example of a wirelessly-configured surgical camera is disclosed in U.S. Patent Application Serial No. 13/025,636. Surgical camera assembly 10 may be wirelessly-configured similarly as described in the 13/025,636 Application.

With reference now to Fig. 3, visualization goggles 400 are configured to be worn by the surgeon and generally include a headset 410 and a support strap 420 configured to secure headset 410 on a surgeon's head. Headset 410 is positionable on the surgeon's face and includes a lens 412, at least a portion of which is transparent so as not to substantially hinder the surgeon's view when wearing visualization goggles 400. Headset 410 is configured to receive the electrical signals from control circuitry 240 of camera 200 and the control circuitry (not explicitly shown) of camera 300 and to display each of the signals as video images. The video images from cameras 200, 300 are displayed on lens 412 as video images "V1" and "V2," respectively. Control electronics (not explicitly shown) within headset 410 may be used to reposition the images "V1" and "V2" on lens 412 as desired for optimal viewing by the surgeon and/or to make the images "V1" and "V2" transparent so as to permit the surgeon to maintain visualization of the surgeon's surroundings through lens 412 in addition to viewing the images "V1" and "V2." As described above, surgical cameras 200, 300 are independent of one another, with each surgical camera 200, 300 producing an image "V1" and "V2," respectively, for display to the surgeon. However, in embodiments where a standard video monitor (not shown) is used in place of visualization goggles 400, control circuitry 240 of camera 200 and the control circuitry (not explicitly shown) of camera 300 may further be configured to multiplex, or synthesize the images produced by each of cameras 200, 300 to provide an electronically synthesized three-dimensional video image for display on the monitor (not shown).

As can be appreciated, with the two-dimensional video images "V1" and "V2" of surgical cameras 200, 300, respectively, displayed to the surgeon adjacent each of the surgeons eyes, a three-dimensional, or stereoscopic view of the internal surgical site "S" (Fig. 4) is created. Surgical camera assembly 10 is configured to produce sharp, reliable three-dimensional imaging of the internal surgical site "S" (Fig. 4) as a result of the fixed positioning of surgical cameras 200, 300 spaced-apart from one another at approximately the same distance as an average human's eyes. More specifically, the image received by each lens 210, 310 is transmitted through legs 110, 120, respectively, base 100, and to headset 410, ultimately to be displayed in front of each of the surgeon's eyes as images "V1" and "V2," respectively. Accordingly, the surgeon is able to view the image "V1" produced by surgical camera 200 through one eye and the image "V2" produced by surgical camera 300 through the other eye, thus simulating the three-dimensional visualization the surgeon would have if the surgeon's eyes were positioned within the internal surgical site "S" (Fig. 4), i.e., at distal ends 114, 124 of legs 110, 120, respectively.

Turning now to Fig. 4, in conjunction with Figs. 1-3 the use and operation of surgical camera assembly 10 is described. Initially, a pair of small punctures, or incisions "I1" and "I2" is formed through tissue "T." Incisions "I1" and "I2" are spaced-apart from one another the distance "D" between legs 110, 120 of surgical camera assembly 10 such that each leg 110, 120 may be inserted through one of the incisions "I1," "I2," respectively, and into the internal surgical site "S," while base 100 of surgical camera assembly 10 remains disposed externally of the internal surgical site "S." Once inserted into the surgical site "S," surgical camera assembly 10 may be manipulated, e.g., tilted, angled, or longitudinally translated, to better position lenses 210, 310 of surgical cameras 200, 300, respectively, within the internal surgical site "S."

Prior to, or once surgical cameras 200, 300 are positioned as desired, the surgeon may put on visualization goggles 400 such that headset 410 is positioned on the surgeon's face and such that lens 412 is positioned directly in front of the surgeon's eyes. Thereafter, surgical cameras 200, 300 can be activated to produce images "V 1" and "V2," as viewed from distal ends 114, 124 of legs 110, 120, respectively, that are displayed on lens 412 of headset 410. As such, the surgeon may perform a surgical task within the internal surgical site "S" (using other surgical instrumentation (not shown)) while visualizing the internal surgical site "S" in three dimensions. As can be appreciated, since cameras 200, 300 are fixed on legs 110, 120, respectively, and, thus, are fixed in position relative to one another, distortion and/or other similar imaging issues that typically arise during manipulation and/or repositioning of three-dimensional video endoscopes are substantially reduced.

At the completion of the surgical task, or procedure, surgical camera assembly 10 may be withdrawn from incisions "I1" and "I2" formed within tissue "T." Due to the relatively small diameters of legs 110, 120 of surgical camera assembly 10 and, thus, the relatively small incisions "I1" and "I2" required to permit passage of legs 110, 120, respectively, therethrough, incisions "I1" and "I2" need not be sutured after removal of surgical camera assembly 10, but may simply be bandaged and allowed to heal naturally.

From the foregoing and with reference to the various figure drawings, those skilled in the art will appreciate that certain modifications can also be made to the present disclosure without departing from the scope of the same. While several embodiments of the disclosure have been shown in the drawings, it is not intended that the disclosure be limited thereto, as it is intended that the disclosure be as broad in scope as the art will allow and that the specification be read likewise. Therefore, the above description should not be construed as limiting, but merely as exemplifications of particular embodiments. Those skilled in the art will envision other modifications within the scope of the claims appended hereto.

## Claims

1. A surgical camera assembly (10), adapted to be inserted in part through an access opening in a body, comprising:
a base (100);
first and second spaced-apart legs (110, 120) extending distally from the base and configured for positioning within an internal surgical site,
a first surgical camera (200) configured to produce a video image of the area extending distally and radially outwardly from the distal end of the first leg;
a second surgical camera (300) configured to produce a video image of the area extending distally and radially outwardly from the distal end of the second leg, wherein the video image produced by the first surgical camera and the video image produced by the second surgical camera are used in conjunction with one another to provide a three-dimensional video image of the internal surgical site, **characterised in that** the first and second surgical cameras are disposed within the first and second leg respectively and the legs are disposed in parallel orientation relative to one another, each leg defining a proximal end slidingly engaged to the base and a free distal end.

2. The surgical camera assembly according to claim 1, wherein the first and second legs are spaced-apart by a distance of about 63,5 mm (2.5 inches).

3. The surgical camera assembly according to claim 1 or claim 2, wherein each of the legs defines a diameter of about 1mm to about 2mm.

4. The surgical camera assembly according to any preceding claim, further comprising a pair of visualization goggles (400) configured to be worn by a surgeon, the visualization goggles coupled to the base and configured to display the video images produced by each of the first and second cameras.

5. The surgical camera assembly according to claim 4, wherein the visualization goggles display the video image produced by the first camera in front of a first eye of the surgeon and the video image produced by the second camera in front of a second eye of the surgeon.

6. The surgical camera assembly according to claim 4 or claim 5, wherein the visualization goggles are coupled to the base by a cable.

7. The surgical camera assembly according to claim 4 or claim 5, wherein the visualization goggles are wirelessly coupled to the base.

8. The surgical camera assembly according to any preceding claim, wherein each surgical camera includes a lens, an image sensor, and control circuitry.

9. The surgical camera assembly according to any preceding claim, wherein the control circuitry of each of the surgical cameras is disposed within the base.

## Patentansprüche

1. Chirurgische Kameraanordnung (10), angepasst dafür, teilweise durch eine Zugangsöffnung in einen Körper eingeführt zu werden, umfassend:
eine Basis (100);
erste und zweite voneinander beabstandete Beine (110, 120), die sich distal von der Basis erstrecken und die zum Positionieren in einer inneren Operationsstelle eingerichtet sind,
eine erste chirurgische Kamera (200), die dafür eingerichtet ist, ein Videobild des Bereichs, der sich distal und radial nach außen von dem distalen Ende des ersten Beins erstreckt, zu erzeugen;
eine zweite chirurgische Kamera (300), die dafür eingerichtet ist, ein Videobild des Bereichs, der sich distal und radial nach außen von dem distalen Ende des zweiten Beins erstreckt, zu erzeugen, wobei das Videobild, das durch die ersten chirurgische Kamera erzeugt wird, und das Videobild, das durch die zweite chirurgische Kamera erzeugt wird, in Verbindung miteinander verwendet werden, um ein dreidimensionales Videobild der inneren Operationsstelle bereitzustellen, **dadurch gekennzeichnet, dass** die ersten und zweiten chirurgischen Kameras entsprechend in den ersten und zweiten Beinen angeordnet sind und die Beine in paralleler Orientierung relativ zueinander angeordnet sind, wobei jedes Bein ein proximales Ende, das gleitbeweglich an der Basis angebracht ist, und ein freies distales Ende definiert.

2. Chirurgische Kameraanordnung gemäß Anspruch 1, bei der die ersten und zweiten Beine durch einen Abstand von ungefähr 63,5 mm (2,5 Zoll) voneinander beabstandet sind.

3. Chirurgische Kameraanordnung gemäß Anspruch 1 oder Anspruch 2, bei der jedes der Beine einen Durchmesser von ungefähr 1 mm bis ungefähr 2 mm definiert.

4. Chirurgische Kameraanordnung gemäß einem der vorhergehenden Ansprüche, die außerdem eine Visualisierungsbrille (400) umfasst, die dafür eingerichtet ist, von einem Chirurg getragen zu werden, wobei die Visualisierungsbrille mit der Basis gekoppelt ist und dafür eingerichtet ist, die Videobilder anzuzeigen, die von jeder der ersten und zweiten Kameras erzeugt werden.

5. Chirurgische Kameraanordnung gemäß Anspruch 4, bei der die Visualisierungsbrille das Videobild, das durch die ersten Kamera erzeugt wird, vor einem ersten Auge des Chirurgen, und das Videobild, das durch die zweite Kamera erzeugt wird, vor einem zweiten Auge des Chirurgen anzeigt.

6. Chirurgische Kameraanordnung gemäß Anspruch 4 oder Anspruch 5, bei der die Visualisierungsbrille durch ein Kabel mit der Basis gekoppelt ist.

7. Chirurgische Kameraanordnung gemäß Anspruch 4 oder Anspruch 5, bei der die Visualisierungsbrille drahtlos mit der Basis gekoppelt ist.

8. Chirurgische Kameraanordnung gemäß einem der vorhergehenden Ansprüche, bei der jede chirurgische Kamera eine Linse, einen Bildsensor und einen Steuerschaltkreis beinhaltet.

9. Chirurgische Kameraanordnung gemäß einem der vorhergehenden Ansprüche, bei der der Steuerschaltkreis jeder der chirurgischen Kameras in der Basis angeordnet ist.

## Revendications

1. Ensemble de caméras chirurgicales (10) qui est à même d'être inséré en partie à travers une ouverture d'accès corporelle, comprenant :
- une base (100) ;
- une première et une seconde jambe (110, 120) espacées l'une de l'autre, s'étendant de manière distale depuis la base et configurées pour se positionner dans un site chirurgical interne,
- une première caméra chirurgicale (200) configurée pour produire une image vidéo de la zone s'étendant de manière distale radialement vers l'extérieur depuis l'extrémité distale de la première jambe ;
- une seconde caméra chirurgicale (300) configurée pour produire une image vidéo de la zone s'étendant de manière distale radialement vers l'extérieur depuis l'extrémité distale de la seconde jambe, dans lequel l'image vidéo produite par la première caméra chirurgicale et l'image vidéo produite par la seconde vidéo chirurgicale sont utilisées conjointement l'une avec l'autre pour fournir une image vidéo tridimensionnelle du site chirurgical interne, **caractérisé en ce que** les première et seconde caméras chirurgicales sont disposées dans la première et la seconde jambe, respectivement, et les jambes sont disposées en orientation parallèle l'une par rapport à l'autre, chaque jambe définissant une extrémité proximale engagée à coulissement sur la base et une extrémité distale libre.

2. Ensemble de caméras chirurgicales selon la revendication 1, dans lequel la première et la seconde jambe sont espacées l'une de l'autre d'environ 63,5 mm (2,5 pouces).

3. Ensemble de caméras chirurgicales selon la revendication 1 ou la revendication 2, dans lequel chacune des jambes définit un diamètre d'environ 1 mm à environ 2 mm.

4. Ensemble de caméras chirurgicales selon l'une quelconque des revendications précédentes, comprenant en outre une paire de lunettes de visualisation (400) configurées pour être portées par un chirurgien, les lunettes de visualisation étant couplées à la base et configurées pour afficher les images vidéo produites par chacune des première et seconde caméras.

5. Ensemble de caméras chirurgicales selon la revendication 4, dans lequel les lunettes de visualisation affichent l'image vidéo produite par la première caméra devant un premier oeil du chirurgien et l'image vidéo produite par la seconde caméra devant un second oeil du chirurgien.

6. Ensemble de caméras chirurgicales selon la revendication 4 ou la revendication 5, dans lequel les lunettes de visualisation sont couplées à la base par un câble.

7. Ensemble de caméras chirurgicales selon la revendication 4 ou la revendication 5, dans lequel les lunettes de visualisation sont couplées à la base sans fil.

8. Ensemble de caméras chirurgicales selon l'une quelconque des revendications précédentes, dans lequel chaque caméra chirurgicale comprend une lentille, un capteur d'image et une circuiterie de commande.

9. Ensemble de caméras chirurgicales selon l'une quelconque des revendications précédentes, dans lequel la circuiterie de commande de chacune des caméras chirurgicales est disposée dans la base.
